# EUROPÄISCHE PATENTSCHRIFT

(11) **EP 3 858 269 B1**
(45) Veröffentlichungstag und Bekanntmachung des Hinweises auf die Patenterteilung: **26.07.2023**
(21) Anmeldenummer: 20202721.5
(22) Anmeldetag: 20.10.2020
(51) Int. Cl.: A61B 17/34, A61B 10/00, A61B 90/00

(54) **KANÜLE, INSBESONDERE FÜR DIE LUMBALPUNKTION UND -INJEKTION, SOWIE HERSTELLUNGSVERFAHREN HIERFÜR**
CANNULA, IN PARTICULAR FOR LUMBAR PUNCTURE AND INJECTION, AND METHOD FOR ITS MANUFACTURE
CANULE, EN PARTICULIER POUR LA PONCTION ET L'INJECTION LOMBAIRES, AINSI QUE PROCÉDÉ DE FABRICATION CORRESPONDANT

(30) Priorität: 29.01.2020 DE 102020102119
(43) Veröffentlichungstag der Anmeldung: 04.08.2021
(73) Patentinhaber: Pajunk GmbH Medizintechnologie, 78187 Geisingen (DE)
(72) Erfinder: Pajunk-Schelling, Simone, 78187 Geisingen (DE); Hauger, Martin, 78166 Donaueschingen (DE)
(74) Vertreter: Westphal, Mussgnug & Partner Patentanwälte mbB

(56) Entgegenhaltungen:
- CN-U- 209 499 970
- DE-A1- 3 020 926
- DE-A1- 4 038 952
- JP-A- 2008 194 112
- US-A1- 2014 324 012

## Beschreibung

Die Erfindung betrifft eine verbesserte Kanüle, insbesondere für die Lumbalpunktion und -injektion mit den Merkmalen des Patentanspruchs 1 sowie ein Herstellungsverfahren einer verbesserten Kanüle mit den Merkmalen des Anspruchs 11.

Aus dem Stand der Technik sind Kanülen in unterschiedlichen Ausgestaltungen vorbekannt, welche typischerweise ein proximales und ein distales Ende und ein Kanülenrohr aufweisen, wobei an dem distalen Ende eine Öffnung vorgesehen ist.

In der Spinalanästhesie werden zur Nervenblockade Betäubungsmittel mittels gattungsgemäßer Kanülen in den Spinalkanal der Wirbelsäule injiziert. Zur Diagnose bestimmter Krankheiten kann durch eine Lumbalpunktion Liquor dem Spinalkanal entnommen oder es können Röntgenkontrastmittel oder Medikamente in den Spinalkanal injiziert werden.

Alzheimer und auch Demenz können durch Vorsorgeuntersuchungen frühzeitig erkannt werden und durch entsprechende Medikamente bei einer rechtzeitigen Diagnose erfolgsversprechend behandelt werden. Hierzu ist es jedoch erforderlich, dass regelmäßige Vorsorgeuntersuchungen durchgeführt werden, bei denen zur Diagnose Liquor aus dem Spinalkanal entnommen wird.

Für diese Verwendungen haben sich in der Vergangenheit Kanülen bewährt, welche eine geschlossene und konische Spitze aufweisen, deren Öffnung für die Entnahme oder Injektion sich seitlich hinter der Spitze befindet. Diese Kanülen sind aus dem Stand der Technik auch als Sprotte^{®}-Kanülen aus der DE 3 020 926 A1 bekannt und zeichnen sich dadurch aus, dass bei der Punktion oder Injektion in die harte Hirnhaut (bzw. Dura mater) des Spinalkanals ein möglichst atraumatischer Einstich und somit ein minimales Punktionsloch geschaffen wird, wodurch ein ungewünschter Austritt von Liquor minimiert werden kann. Ein Verlust an Liquor kann zu einem Druckabfall im gesamten Liquorraum und dadurch zu einer verbreiteten Nebenwirkung, wie beispielsweise Kopfschmerzen, führen. Ein starker Druckabfall kann sogar zu vorübergehenden Lähmungen von Hirnnerven oder zu Rissen in den Blutgefäßen der Hirnhäute führen.

Alternativ können Kanülen verwendet werden, deren Spitze als distalen Ende durch einen Schrägschliff des Kanülenrohrs ausgebildet wird.

Als weiterer Stand der Technik werden die US 2014/324012 A1, die JP 2008 194112 A, die DE 40 38 952 A1 sowie die CN 209 499 970 U genannt.

Als ein Nachteil der aus dem Stand der Technik bekannten Kanülen ist zu nennen, dass bei der Herstellung der aus dem Stand der Technik bekannten Kanülen für die Spinalpunktion eine äußerst umfangreiche Reinigung erforderlich ist, um sicherzustellen, dass nach dem Herstellungsprozess keine Verunreinigungen sowohl auf dem Kanülenrohr, aber insbesondere auch in dem Kanülenrohr, zurückbleiben.

Hier setzt die vorliegende Erfindung an.

Es ist daher die Aufgabe der vorliegenden Erfindung, eine verbesserte Kanüle für die Lumbalpunktion und -injektion bereitzustellen, die die aus dem Stand der Technik bekannten Kanülen zweckmäßigerweise verbessert und durch ein Herstellungsverfahren bereitgestellt werden kann, welches bei gleicher oder verbesserter Funktionalität der Kanüle wirtschaftliche Vorteile in sich vereint. Die verbesserte Kanüle soll insbesondere die Patientensicherheit erhöhen.

Diese Aufgaben werden durch eine Kanüle mit den Merkmalen des Patentanspruchs 1 und ein Verfahren zum Herstellen einer Kanüle für die Lumbalpunktion und -injektion mit den Merkmalen des Patentanspruchs 11 gelöst.

Weitere vorteilhafte Ausgestaltungen der vorliegenden Erfindung werden in den Unteransprüchen angegeben.

Die erfindungsgemäße Kanüle, insbesondere für die Lumbalpunktion und -injektion mit den Merkmalen des Patentanspruchs 1 hat ein proximales Ende und ein distales Ende und weist weiterhin ein Kanülenrohr mit einer Innenseite, einer Außenseite, einem ersten Endbereich und einem zweiten Endbereich auf, wobei das Kanülenrohr einen Kanülenkanal bildet. Darüber hinaus weist das Kanülenrohr an dem zweiten Endbereich eine Spitze auf, die das distale Ende der Kanüle bildet, wobei in dem Kanülenrohr eine Öffnung des Kanülenkanals vorgesehen ist und wobei in dem Kanülenkanal auf der Innenseite zumindest um die Öffnung eine Beschichtung vorgesehen ist.

Die Kernidee der vorliegenden Erfindung basiert auf dem Gedanken, dass nach dem Einbringen der Öffnung, welche beispielsweise durch ein spanendes Verfahren, durch ein Erosionsverfahren oder durch Laserschneiden hergestellt werden kann, eine Beschichtung wenigstens um die Öffnung auf die Innenseite des Kanülenrohrs aufgebracht wird. Die Beschichtung kann sicherstellen, dass selbst nach einem technischen Reinigen der Innenseite des Kanülenrohrs zurückbleibende Verunreinigungen bzw. Rückstände eingebunden werden, wodurch die Kanüle für die durchzuführenden Untersuchungen besonders rein ist und die Patientensicherheit gegeben ist. Weiterhin verhindert die Beschichtung, dass sich Material, beispielsweise in Form von Metallionen, aus dem Kanülenrohr lösen kann und in die durch das Kanülenrohr strömende Flüssigkeit eintritt. Zudem kann die Beschichtung eine Optimierung und/oder Egalisierung der Oberfläche erreichen.

Erfindungsgemäß überdeckt die Beschichtung eine Innenkante der Öffnung. Typischerweise wird die Öffnung in das Kanülenrohr durch das Herstellungsverfahren Schleifen eingearbeitet und es hat sich gezeigt, dass die Beschichtung der Innenkante sowohl Verschmutzungen einschließen kann als auch fertigungsbedingte Rauheiten und scharfkantige Bereiche reduziert werden können. Weiterhin ist die Beschichtung auf der Außenseite zumindest um die Öffnung angeordnet. Erfindungsgemäß ist die Beschichtung auf der Innenseite des Kanülenrohrs wenigstens um die Öffnung und die Beschichtung auf der Außenseite des Kanülenrohrs wenigstens um die Öffnung herum durch die Öffnung über die Innenkante miteinander verbunden sind, wodurch eine einstückige in sich geschlossene Beschichtung vorgesehen sein kann, die sämtliche Verunreinigungen im Bereich der Öffnung einschließen kann.

Weiterhin ist erfindungsgemäß vorgesehen, dass die Innenseite und die Außenseite des Kanülenrohrs vollständig durch die Beschichtung bedeckt sind. Erfindungsgemäß weist die Beschichtung eine Dicke im Bereich von 0,1 µm bis 50 µm auf.

Die Schichtdicke der Beschichtung kann über die einzelnen Bereiche des Kanülenrohrs variieren, wobei die Schichtdicke sowohl auf der Innenseite als auch auf der Außenseite des Kanülenrohrs möglichst dünn gehalten werden sollte, um den Durchmesser des Kanülenrohrs auf der Außenseite nicht unnötigerweise zu vergrößern und auf der Innenseite nicht zu verkleinern. Weiterhin sollte ein in dem Kanülenrohr durchströmbarer Querschnitt ausreichend groß bemessen sein, damit auch ein Mandrin in dem Kanülenrohr eingesetzt werden kann, um beim Einbringen der Kanüle das Eindringen von Gewebe in das Kanülenrohr zu verhindern.

Gemäß einer bevorzugten Weiterbildung der Erfindung umfasst die Kanüle einen Mandrin, welcher ebenfalls zumindest abschnittsweise, vorzugsweise vollständig mit einer Beschichtung versehen ist.

Vorzugsweise ist die Spitze geschlossen und annäherungsweise konisch ausgebildet und die Öffnung benachbart zu der Spitze das Kanülenrohrs angeordnet. Durch eine derartige Ausgestaltung kann eine atraumatische Kanüle bereitgestellt werden, bei welcher vermieden werden kann, dass beim Einstechen Gewebe in die Öffnung eindringt.

Nach Maßgabe einer weiteren vorteilhaften Ausgestaltung der vorliegenden Kanüle kann die Öffnung in einer Längsrichtung des Kanülenrohrs langgezogenen sein. Die Längsrichtung des Kanülenrohrs wird durch die Richtung der längsten Erstreckung des Kanülenrohrs vorgegeben, mit anderen Worten durch den Vektor zwischen dem distalen Ende und dem proximalen Ende des Kanülenrohrs. Unter einer langgezogenen Öffnung des Kanülenrohrs wird hier und im nachfolgenden eine Öffnung verstanden, die in der Längsrichtung länger ist als quer zu der Längsrichtung, vorzugsweise mindestens in einem Verhältnis 1,5:1.

Eine alternative bevorzugte Weiterbildung der Erfindung sieht vor, dass das Kanülenrohr an dem zweiten Endbereich einen Schrägschliff aufweist, durch welchen die Spitze gebildet ist, und die Öffnung stirnseitig in dem zweiten Endbereich an dem Kanülenrohr angeordnet ist.

Eine weitere vorteilhafte Ausgestaltung der vorliegenden Erfindung sieht vor, dass am proximalen Ende der Kanüle ein Ansatz vorgesehen ist. Der Ansatz kann einen Anschluss aufweisen, durch den der Ansatz mit einem Schlauch oder einer Spritze verbunden werden kann. Vorzugsweise kann der Anschluss beispielsweise als Konnektor, insbesondere als Luer-Lock-Konnektor oder als NRFit^{®}-Konnektor ausgebildet sein.

Weiterhin hat es sich als vorteilhaft erwiesen, dass die Beschichtung ebenfalls den Ansatz ummantelt. Die Beschichtung kann in einer bevorzugten Ausgestaltung der vorliegenden Erfindung die Kanüle vollständig umgeben, sodass sowohl die Innenseite als auch alle äußeren Oberflächen der Kanüle mit einer Beschichtung versehen sind, wodurch sämtliche Verunreinigungen eingeschlossen werden.

Vorzugsweise weist das Kanülenrohr einen Außendurchmesser im Bereich von 0,2 mm bis 1,5 mm, vorzugsweise im Bereich von 0,4 mm bis 1,2 mm, einen Innendurchmesser im Bereich von 0,15 mm bis 1,4 mm und/oder eine Länge im Bereich von 90 mm bis 500 mm, vorzugsweise von 90 bis 300 mm, besonders bevorzugt von 90 mm bis 120 mm aufweist.

Die Beschichtung weist vorzugsweise eine Dicke im Bereich von 0,1 µm bis 30 µm, besonders bevorzugt im Bereich von 0,1 µm bis 15 µm, auf.

Ein weiterer Aspekt der vorliegenden Erfindung betrifft ein Herstellungsverfahren einer Kanüle, insbesondere einer erfindungsgemäßen Kanüle für die Lumbalpunktion und -injektion, wobei das erfindungsgemäße Herstellungsverfahren folgende Verfahrensschritte aufweist:
- Bereitstellen des Kanülenrohrs mit einer Innenseite, einer Außenseite, einem offenen ersten Endbereich und einem zweiten Endbereich,
- Einbringen einer Öffnung, und
- Beschichten der Innenseite des Kanülenrohrs zumindest um die Öffnung.

Vorzugsweise ist vorgesehen, dass entweder der zweite Endbereich eine Spitze aufweist, welche geschlossen und annäherungsweise konisch ausgebildet ist, und die Öffnung benachbart zu der Spitze eingebracht wird, oder dass das Kanülenrohr an dem zweiten Endbereich mit einem Schrägschliff bearbeitet wird, durch welchen eine Spitze gebildet wird, und die Öffnung stirnseitig in dem zweiten Endbereich an dem Kanülenrohr angeordnet ist.

Erfindungsgemäß ist vorgesehen, dass die gesamte Innenseite und die gesamte (frei liegende) Außenseite des Kanülenrohrs beschichtet werden. Unter der frei liegenden Außenseite des Kanülenrohrs wird dabei der Bereich des Kanülenrohrs verstanden, der beispielsweise nicht durch den Ansatz verdeckt ist.

Eine vorteilhafte Weiterbildung des vorliegenden Verfahrens sieht vor, dass die Öffnung in das Kanülenrohr durch ein spannendes Herstellungsverfahren, insbesondere durch Schleifen, alternativ durch Erodieren oder Laserschneiden, eingearbeitet wird. Durch diese Herstellungsverfahren kann die Öffnung in das Kanülenrohr eingearbeitet werden, ohne dass die Gefahr besteht, dass ungewünschte Verformungen an dem Kanülenrohr entstehen.

Darüber hinaus hat es sich als vorteilhaft erwiesen, wenn vor dem Beschichten ein Ansatz an den ersten Endbereich auf das Kanülenrohr aufgeformt wird, wobei vorzugsweise der Ansatz durch Spritzgießen auf das Kanülenrohr aufgeformt wird.

Es kann insbesondere vorteilhaft sein, wenn ein distales und geschlossenes Ende des Kanülenkanals bzw. das "tote" Ende in dem Kanülenkanal in dem zweiten Endbereich des Kanülenrohrs zwischen der Öffnung und dem geschlossenen zweiten Endbereich verschlossen wird.

Erfindungsgemäß wird die Innenkante der Öffnung beschichtet und weiterhin wenigstens die Außenseite des Kanülenrohrs um die Öffnung herum. In einer besonders bevorzugten Ausgestaltung der vorliegenden Erfindung werden alle Oberflächen der Kanüle nach dem Einbringen der Öffnung mit der Beschichtung versehen, sodass sämtliche möglichen Verunreinigungen an der Kanüle eingeschlossen sind und eine besonders reine Kanüle bereitgestellt werden kann.

Ein weiterer Aspekt der vorliegenden Erfindung betrifft die Verwendung einer erfindungsgemäßen Kanüle für die Lumbalpunktion und -injektion.

Nachfolgend wird unter Bezugnahme auf die begleitenden Zeichnungen ein Ausführungsbeispiel einer erfindungsgemäßen Kanüle, insbesondere für die Lumbalpunktion und -injektion, im Detail beschrieben. Es zeigen:
- Figur 1a: eine schematische, teilweise geschnittene und stark vereinfachte Darstellung eines ersten Ausführungsbeispiels einer Kanüle für die Lumbalpunktion und -injektion,
- Figur 1b: eine vergrößerte Darstellung eines distalen Endes der Kanüle mit einer konischen und geschlossenen Spitze,
- Figur 2: eine schematische, teilweise geschnittene und stark vereinfachte Darstellung eines Mandrins einer Kanüle für die Lumbalpunktion und -injektion gemäß Figur 1,
- Figur 3a: eine schematische, teilweise geschnittene und stark vereinfachte Darstellung eines zweiten Ausführungsbeispiels einer Kanüle für die Lumbalpunktion und-injektion,
- Figur 3b: eine vergrößerte Darstellung eines distalen Endes der Kanüle mit einer durch einen Schrägschliff gebildeten Spitze,
- Figur 4: eine schematische, teilweise geschnittene und stark vereinfachte Darstellung eines Mandrins einer Kanüle für die Lumbalpunktion und -injektion gemäß Figur 3.

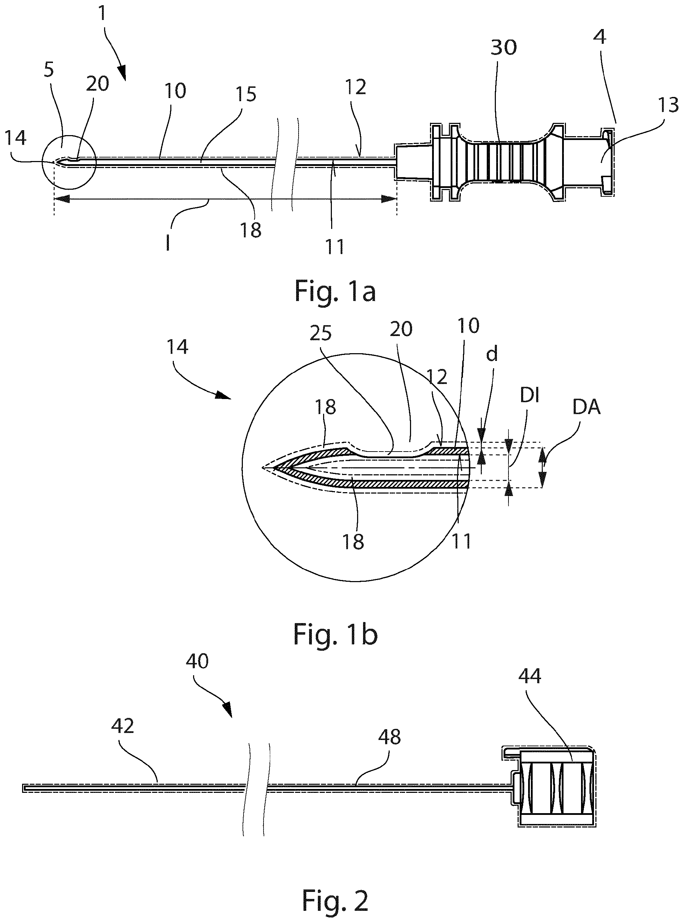

Figur 1a zeigt eine erfindungsgemäße Kanüle 1 für die Lumbalpunktion und -injektion, welche dazu verwendet werden kann, ein Betäubungsmittel in den Spinalkanal einer Wirbelsäule eines Patienten zu injizieren. Weiterhin kann die in Figur 1a dargestellte Kanüle 1 verwendet werden, zur Diagnose oder zu Vorsorgezwecken Liquor aus dem Spinalkanal eines Patienten zu entnehmen, oder ein Röntgenkontrastmittel in den Spinalkanal zu injizieren.

Die Kanüle 1 hat ein proximales Ende 4 und ein distales Ende 5 und umfasst ein Kanülenrohr 10 und einen Ansatz 30.

Das Kanülenrohr 10 kann aus einem metallischen Werkstoff oder einen Kunststoff, beispielsweise einem kohlefaserverstärkten Kunststoff, hergestellt sein. Das Kanülenrohr 10 kann einen Außendurchmesser DA im Bereich von 0,2 mm bis 1,5 mm, vorzugsweise im Bereich von 0,4 mm bis 1,2 mm aufweisen. Weiterhin kann das Kanülenrohr 10 einen Innendurchmesser DI im Bereich von 0,15 mm bis 1,4 mm aufweisen. Schließlich kann das Kanülenrohr 10 eine Länge 1 im Bereich von 90 mm bis 500 mm, vorzugsweise von 90 bis 300 mm, besonders bevorzugt von 90 mm bis 120 mm aufweisen.

Das Kanülenrohr 10 weist einen offenen ersten Endbereich und gemäß dem in den Figuren 1a und 1b dargestellten ersten Ausführungsbeispiel einen geschlossenen zweiten Endbereich auf. Das Kanülenrohr 10 weist eine Innenseite 11 und eine Außenseite 12 auf, wobei die Innenseite 11 die Wandung eines Lumens bzw. des Kanülenkanals 15 bildet. Der zweite Endbereich des Kanülenrohrs 10 weist eine konische oder kegelförmige Spitze 14 auf, die das distale Ende 5 der Kanüle 1 bildet.

Benachbart zu der Spitze 14 des Kanülenrohrs 10, welche in Detail in Figur 1b dargestellt ist, ist eine Öffnung 20 mit einer die Öffnung 20 umgebenden Innenkante 25 angeordnet, durch die der Kanülenkanal 15 aus einer Radialrichtung zugänglich ist. Ein Abstand zwischen der Öffnung 20 und dem distalen Ende 4 bzw. der Spitze 14 ist derart gewählt, dass die Öffnung 20 vollständig auf einer Mantelfläche des Kanülenrohrs 10 ausgebildet ist.

An dem proximalen Ende 4 ist auf dem Kanülenrohr 10 der Ansatz 30 aufgeformt, wobei bevorzugt der Ansatz 30 einen Anschluss ausbildet, an dem ein Schlauch oder eine Spritze befestigt werden kann. Der Anschluss kann als Luer-Lock-Konnektor oder als NRFit^{®}-Konnektor ausgebildet sein.

Wie den Figuren 1a und 1b zu entnehmen ist, weist die Kanüle 1 eine durch gestrichelte Linien dargestellte Beschichtung 18 auf. Die Beschichtung 18 ist auf der Innenseite 11 und/oder der Außenseite 12 des Kanülenrohrs 10 um die Öffnung 20 herum aufgetragen und verschließt den Kanülenkanal 15 zwischen der Öffnung 20 und dem distalen Ende 5. Weiterhin kann die Beschichtung 18 - wie dargestellt ist - sich vollständig über die Innenkante 25 der Öffnung 20 erstrecken, wodurch eine einstückige oder monolithische Beschichtung 18 gebildet werden kann. Bevorzugt bedeckt die Beschichtung vollständig und ununterbrochen sämtliche Flächen des Kanülenrohrs 10 bzw. der Kanüle 1 und schließt vorzugsweise mögliche Verunreinigungen auf den Flächen ein, wodurch diese unschädlich gemacht werden. Die Beschichtung 18 ist bevorzugt diffusionsdicht und/oder abrasionsfest.

Zum Herstellen der Kanüle gemäß Figur 1 wird zunächst das Kanülenrohr 10 bevorzugt vereinzelt bereitgestellt und anschließend kann auf den offenen ersten Endbereich des Kanülenrohrs 10 der Ansatz 30, beispielsweise durch Spritzgießen, aufgeformt werden. In einem darauffolgenden Schritt kann die Öffnung 20 in das Kanülenrohr 10 in dem zweiten Endbereich benachbart zu der Spitze 14 eingearbeitet werden, wobei bevorzugt die Öffnung 20 als eine langgestreckte Öffnung 20 in das Kanülenrohr 10 durch ein spanendes Fertigungsverfahren, insbesondere Schleifen, eingebracht werden kann.

Nach dem Einarbeiten der Öffnung in das Kanülenrohr 10 kann die bereits funktionsfähige Kanüle 1 durch ein Reinigungsverfahren gereinigt werden, um Verunreinigungen durch das Einbringen der Öffnung 20 und die anderen Herstellungsschritte zu entfernen. Um sicherzustellen, dass auch nach dem Reinigen insbesondere an schwer zugänglichen Stellen wie beispielsweise innerhalb der geschlossenen und konischen Spitze 14 an dem distalen Ende 5 der Kanüle 1 oder in einem "toten" Ende zwischen der Öffnung 20 und dem geschlossenen zweiten Endbereich des Kanülenrohrs 10 keine Verunreinigungen zurückbleiben, kann eine Beschichtung 18, welche in den begleitenden Figuren als gestrichelte Linien dargestellt ist, vollflächig sowohl auf alle Innenflächen als auch Außenflächen aufgetragen werden.

Die Beschichtung 18 kann sämtliche Verunreinigungen einschlie-βen oder verkleben, die sowohl in dem Kanülenrohr 10 als auch außen auf dem Kanülenrohr 10 und auf dem Ansatz angeordnet sein können.

Die Beschichtung 18 kann vorzugsweise mit einem Dünnschichtverfahren aufgetragen werden und kann sowohl aus einem Kunststoff als auch aus einem metallischen Werkstoff gebildet sein. Eine Dicke d der Beschichtung kann im Bereich von 0,1 µm bis 50 µm, vorzugsweise im Bereich von 0,1 µm bis 30 µm, besonders bevorzugt im Bereich von 0,1 µm bis 15 µm, liegen.

Figur 2 zeigt einen Mandrin 40, aufweisend einen Stababschnitt 42 und einen Ansatzabschnitt 44, wobei der Stababschnitt 32 in das Lumen bzw. den Kanülenkanal 15 einführbar ist und das Kanülenrohr 10 im eingesteckten Zustand stabilisiert. Der Mandrin 40 kann beim Einstich die Öffnung 20 verschließen und verhindert das Eindringen von Gewebe, welches das Kanülenrohr 15 verstopfen könnte. Der Mandrin 40, insbesondere der Stababschnitt 42 kann einen metallischen Kern oder einen Kern aus einem Kunststoff, beispielsweise einem kohlefaserverstärkten Kunststoff, aufweisen, währenddessen der Ansatzabschnitt 44 analog zu dem Ansatz 30 der Kanüle 1 auf den Kern aufgeformt sein kann. Der Mandrin 40 kann weiterhin eine Beschichtung 48 aufweisen, die den Mandrin 40 vollumfänglich abdeckt. Der Werkstoff und das Beschichtungsverfahren der Beschichtungen 18, 48 der Kanüle 1 und des Mandrins 40 können identisch sein.

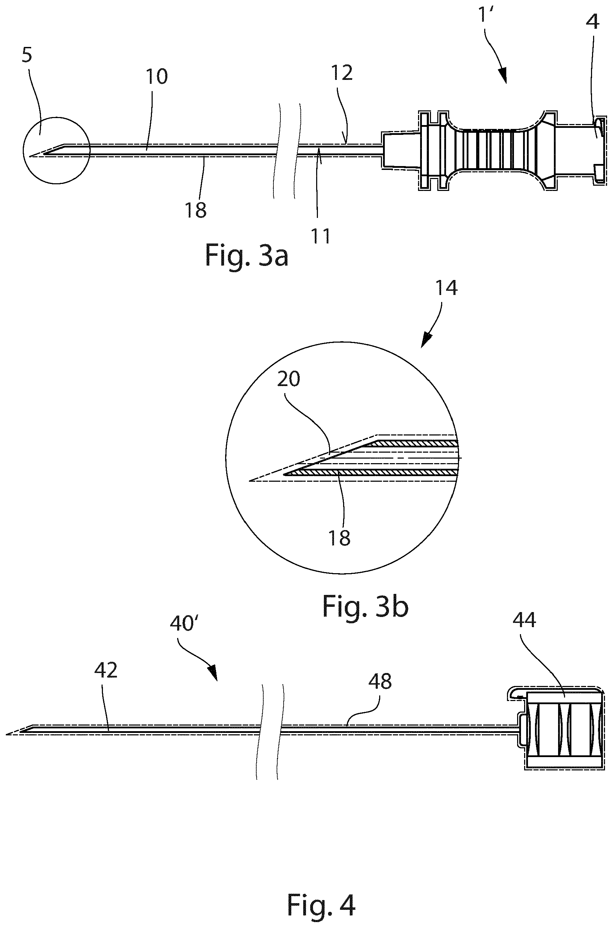

Das in den Figuren 3a und 3b dargestellte zweite Ausführungsbeispiel einer Kanüle 1` sowie das in den Figuren 4 dargestellte zweite Ausführungsbeispiel eines Mandrins 40` unterscheidet sich von der in den Figuren 1a und 1b dargestellten Kanüle 1 bzw. dem in Figur 2 dargestellten Mandrin 40 lediglich in der Ausgestaltung des distalen Endes. Die sonstigen Ausführungen zur Kanüle 1 und dem Mandrin 40 sind auf die Kanüle 1` und den Mandrin 40' übertragbar.

Das Kanülenrohr 10 weist einen offenen ersten Endbereich und einen offenen zweiten Endbereich auf. Der zweite Endbereich des Kanülenrohrs 10 weist dabei einen Schrägschliff auf, durch welchen die Spitze 14 gebildet ist, die das distale Ende 5 der Kanüle 1 bildet. Die Öffnung 20 ist in diesem Ausführungsbeispiel nicht auf der Mantelfäche des Kanülenrohrs 10, sondern stirnseitig in dem zweiten Endbereich des Kanülenrohrs 10 ausgebildet.

Auch die Kanüle 1` weist eine durch gestrichelte Linien dargestellte Beschichtung 18 auf. Die Beschichtung 18 ist auf der Innenseite 11 und/oder der Außenseite 12 des Kanülenrohrs 10 um die Öffnung 20 herum aufgetragen. Weiterhin kann die Beschichtung 18 - wie dargestellt ist - sich vollständig über die Innenkante 25 der Öffnung 20 erstrecken, wodurch eine einstückige oder monolithische Beschichtung 18 gebildet werden kann. Bevorzugt bedeckt die Beschichtung vollständig und ununterbrochen sämtliche Flächen des Kanülenrohrs 10 bzw. der Kanüle 1.

Auch die Kanüle 1` kann einen Mandrin 40' umfassen, welcher in Figur 4 dargestellt ist und sich von dem in Figur 2 dargestellten Mandrin 40 lediglich dadurch unterscheidet, dass ein distales Ende des Mandrins 40' abgeschrägt ausgebildet ist.

### Bezugszeichenliste

- 1, 1': Kanüle
- 4: proximales Ende
- 5: distales Ende
- 10: Kanülenrohr
- 11: Innenseite
- 12: Außenseite
- 14: Spitze
- 15: Kanülenkanal
- 20: Öffnung
- 25: Innenkante
- 30: Ansatz
- 40, 40': Mandrin
- 42: Stababschnitt
- 44: Ansatzabschnitt
- 48: Beschichtung
- d: Dicke
- DA: Außendurchmesser
- DI: Innendurchmesser
- l: Länge

## Patentansprüche

1. Kanüle (1, 1'), insbesondere für die Lumbalpunktion und
- injektion, mit einem proximalen Ende (4) und einem distalen Ende (5), aufweisend
- ein Kanülenrohr (10) mit einer Innenseite (11), einer Außenseite (12), einem ersten Endbereich und einen zweiten Endbereich, welches einen Kanülenkanal (15) bildet,
- wobei das Kanülenrohr (10) an dem zweiten Endbereich eine Spitze (14) aufweist, welche das distale Ende (5) der Kanüle (1) bildet,
- wobei das Kanülenrohr (10) eine Öffnung (20) des Kanülenkanals (15) aufweist, und
- wobei zumindest in dem Kanülenkanal (15) auf der Innenseite (11) um die Öffnung (20) eine Beschichtung (18) vorgesehen ist,
**dadurch gekennzeichnet, dass**
die Beschichtung (18) eine Innenkante (25) der Öffnung (20) überdeckt, dass die Beschichtung (18) auf der Außenseite (12) um die Öffnung (20) angeordnet ist, dass die Beschichtung (18) auf der Innenseite (11) des Kanülenrohrs (10) und auf der Außenseite (12) des Kanülenrohrs (10) durch die Öffnung (20) hindurch verbunden ist, dass die Beschichtung (18) die Innenseite (11) und/oder die Außenseite (12) des Kanülenrohrs (10) vollständig bedeckt und dass die Beschichtung (18) eine Dicke (d) im Bereich von 0,1 µm bis 50 µm aufweist.

2. Kanüle nach Anspruch 1,
**dadurch gekennzeichnet, dass** die Spitze (14) geschlossen und annäherungsweise konisch ausgebildet ist und die Öffnung (20) benachbart zu der Spitze (14) das Kanülenrohrs (10) angeordnet ist.

3. Kanüle nach Anspruch 2,
**dadurch gekennzeichnet, dass**
die Öffnung (20) in einer Längsrichtung des Kanülenrohrs (10) langezogen ist.

4. Kanüle nach Anspruch 1,
**dadurch gekennzeichnet, dass** das Kanülenrohr (10) an dem zweiten Endbereich einen Schrägschliff aufweist, durch welchen die Spitze (14) gebildet ist, und die Öffnung (20) stirnseitig in dem zweiten Endbereich an dem Kanülenrohr (10) angeordnet ist.

5. Kanüle nach einem der vorgenannten Ansprüche,
**dadurch gekennzeichnet, dass**
ein Ansatz (30) am proximalen Ende (4) vorgesehen ist.

6. Kanüle nach einem der vorgenannten Ansprüche,
**dadurch gekennzeichnet, dass**
die Beschichtung (18) den Ansatz (30) ummantelt.

7. Kanüle nach einem der vorgenannten Ansprüche,
**dadurch gekennzeichnet, dass**
die Beschichtung (18) einstückig oder monolithisch ist.

8. Kanüle nach einem der vorgenannten Ansprüche,
**dadurch gekennzeichnet, dass** die Kanüle (1) einen Mandrin (10, 10') umfasst, welcher eine Beschichtung (48) aufweist.

9. Kanüle nach einem der vorgenannten Ansprüche,
**dadurch gekennzeichnet, dass** das Kanülenrohr (10) einen Außendurchmesser (DA) im Bereich von 0,2 mm bis 1,5 mm, vorzugsweise im Bereich von 0,4 mm bis 1,2 mm, einen Innendurchmesser (DI) im Bereich von 0,15 mm bis 1,4 mm und/oder eine Länge (l) im Bereich von 90 mm bis 500 mm, vorzugsweise von 90 bis 300 mm, besonders bevorzugt von 90 mm bis 120 mm aufweist.

10. Kanüle nach einem der vorgenannten Ansprüche,
**dadurch gekennzeichnet, dass** die Beschichtung (18) eine Dicke (d) im Bereich von 0,1 µm bis 30 µm, besonders bevorzugt im Bereich von 0,1 µm bis 15 µm, aufweist.

11. Herstellungsverfahren einer Kanüle (1, 1`), insbesondere für die Lumbalpunktion und -injektion und nach einem der vorgenannten Ansprüche, **gekennzeichnet durch** die Verfahrensschritte:
- Bereitstellen des Kanülenrohrs (10) mit einer Innenseite (11), einer Außenseite (12), mit einem offen ersten Endbereich und einem zweiten Endbereich,
- Einbringen einer Öffnung (20) insbesondere mit einem spanenden Verfahren und
- Beschichten der Innenseite (11) des Kanülenrohrs (10) zumindest um die Öffnung (20)
**dadurch gekennzeichnet**, dass
die gesamte Innenseite (11) und Außenseite (12) des Kanülenrohrs (10) sowie die Innenkante der Öffnung (20) beschichtet wird.

12. Herstellungsverfahren nach Anspruch 11,
**dadurch gekennzeichnet, dass** der zweite Endbereich eine Spitze (14) aufweist, welche geschlossen und annäherungsweise konisch ausgebildet ist, und die Öffnung (20) benachbart zu der Spitze (14) eingebracht wird.

13. Herstellungsverfahren nach Anspruch 11,
**dadurch gekennzeichnet, dass** das Kanülenrohr (10) an dem zweiten Endbereich mit einem Schrägschliff bearbeitet wird, durch welchen eine Spitze (14) gebildet wird, und die Öffnung (20) stirnseitig in dem zweiten Endbereich an dem Kanülenrohr (10) angeordnet ist.

14. Herstellungsverfahren nach einem der Ansprüche 11 bis 13,
**dadurch gekennzeichnet, dass**
vor dem Beschichten ein Ansatz (30) an dem ersten Endbereich auf das Kanülenrohr (10) aufgeformt wird, insbesondere durch Spritzgrießen.

## Claims

1. Cannula (1, 1'), in particular for lumbar puncture and injection, having a proximal end (4) and a distal end (5), comprising
- a cannula tube (10), having an inner side (11), and outer side (12), a first end region and a second end region, which form a cannula channel (15),
- wherein the cannula tube (10) comprises, on the second end region, a tip (14), which forms the distal end (5) of the cannula,
- wherein the cannula tube (10) comprises an opening (20) of the cannula channel (15), and
- wherein, at least in the cannula channel (15), a coating (18) is provided on the inner side (11) around the opening (20)
**characterized in that**
the coating (18) covers an inner edge (25) of the opening (20), **in that** the coating (18) is arranged around the opening (20) on the outer side (12), **in that** the coating (18) on the inner side (11) of the cannula tube (10) and on the outer side (12) of the cannula tube (10) is connected through the opening (20), **in that** the coating (18) completely covers the inner side (11) and/or the outer side (12) of the cannula tube (10), and **in that** the coating (18) a thickness (d) in the range from 0.1 µm to 50 µm.

2. Cannula in accordance with claim 1,
**characterized in that** the tip (14) is closed and is implemented approximately conically and **in that** the opening (20) is arranged adjacently to the tip (14) of the cannula tube (10).

3. Cannula in accordance with claim 2,
**characterized in that** the opening (20) is elongated in a longitudinal direction of the cannula tube (10).

4. Cannula in accordance with claim 1,
**characterized in that** the cannula tube (10) comprises a beveled grinding on the second end region, by means of which the tip (14) is formed, and **in that** the opening (20) is arranged on the channel tube (10) in the second end region on the end face.

5. Cannula in accordance with any of the preceding claims,
**characterized in that** an attachment (30) is provided on the proximal end (4).

6. Cannula in accordance with any of the preceding claims,
**characterized in that** the coating (18) encases the attachment (30).

7. Cannula in accordance with any of the preceding claims,
**characterized in that** the coating (18) is integral or monolithic.

8. Cannula in accordance with any of the preceding claims,
**characterized in that** the cannula (1) includes a stylet (10, 10'), which comprises a coating (48).

9. Cannula in accordance with any of the preceding claims,
**characterized in that** the cannula tube (10) comprises an outer diameter (DA) in the range from 0.2 mm to 1.5 mm, preferably in the range from 0.4 mm to 1.2 mm, an inner diameter (DI) in the range from 0.15 mm to 1.4 mm and/or a length (l) in the range from 90 mm to 500 mm, preferably from 90 to 300 mm, particularly preferably from 90 mm to 120 mm.

10. Cannula in accordance with any of the preceding claims,
**characterized in that** the coating (18) comprises a thickness (d) in the range from 0.1 µm to 30 µm, particularly preferably in the range from 0.1 µm to 15 µm.

11. Manufacturing method for a cannula (1, 1'), in particular for lumbar puncture and injection and in accordance with any of the preceding claims, **characterized by** the method steps:
- providing the cannula tube (10), having an inner side (11), an outer side (12), an open first end region and a second end region,
- creating an opening (20), in particular using a metal-cutting process, and
- coating the inner side (11) of the cannula tube (10), at least around the opening (20)
**characterized in that**
the entire inner side (11) and the outer side (12) of the cannula tube (10), as well as the inner edge of the opening (20), are coated.

12. Manufacturing method in accordance with claim 11,
**characterized in that** the second end region comprises a tip (14) which is closed and which is implemented approximately conically, and **in that** the opening (20) is created adjacent to the tip (14).

13. Manufacturing method in accordance with claim 11,
**characterized in that** the cannula tube (10) is machined on the second end region with a beveled grinding, by means of which a tip (14) is formed, and **in that** the opening (20) is arranged on the cannula tube (10) in the second end region on the end face.

14. Manufacturing method in accordance with any of claims 11 to 13,
**characterized in that**
before coating, an attachment (30) is formed onto the cannula tube (10) at the first end region, in particular by means of injection molding.

## Revendications

1. Canule (1, 1') notamment pour la ponction et l'injection lombaire avec une extrémité proximale (4) et une extrémité distale (5) comprenant :
- un tube de canule (10) avec un côté intérieur (11), un côté extérieur (12), une première zone d'extrémité et une seconde zone d'extrémité formant un canal de canule (15),
- le tube de canule (10) a dans la seconde zone d'extrémité, une pointe (14) formant l'extrémité distale (5) de la canule (1),
- le tube de canule (10) a une ouverture (20) de canal de canule (15), et
- au moins un revêtement (18) est prévu sur le côté intérieur (11) autour de l'orifice (20) dans le canal de canule (15),
canule **caractérisée en ce que**
- le revêtement (18) couvre une arête intérieure (25) de l'ouverture (20),
- le revêtement (18) est prévu sur le côté extérieur (12) autour de l'orifice (20),
- le revêtement (18) est relié sur le côté intérieur (11) du tube de canule (10) et sur le côté extérieur (12) du tube de canal (10) à travers l'ouverture (20),
- le revêtement (18) couvre complètement le côté intérieur (11) et/ou le côté extérieur (12) du tube de canule (10), et
- le revêtement (18) a une épaisseur (d) de l'ordre de 0,1 µm à 50 µm.

2. Canule selon la revendication 1,
**caractérisée en ce que**
la pointe (14) est fermée et elle est sensiblement conique et l'orifice (20) est voisin de la pointe (14) du tube de canule (10).

3. Canule selon la revendication 2,
**caractérisée en ce que**
l'orifice (20) est étiré dans la direction longitudinale du tube de canule (10).

4. Canule selon la revendication 1,
**caractérisée en ce que**
la seconde zone d'extrémité le tube de canal (10) a une coupe en biais qui forme la pointe (14) et l'ouverture (20) est du côté frontal dans la seconde zone d'extrémité du tube de canule (10).

5. Canule selon l'une des revendications précédentes,
**caractérisée en ce que**
une embase (30) est prévue à l'extrémité proximale (4).

6. Canule selon l'une des revendications précédentes,
**caractérisée en ce que**
le revêtement (18) entoure la prolongement (30).

7. Canule selon l'une des revendications précédentes,
**caractérisée en ce que**
le revêtement (18) est en une seule partie ou monolithique.

8. Canule selon l'une des revendications précédentes,
**caractérisée en ce que**
la canule (1) a un mandrin (10, 10') muni d'un revêtement (48).

9. Canule selon l'une des revendications précédentes,
**caractérisée en ce que**
le tube de canal (10) a un diamètre extérieur (DA) dans l'ordre de 0,2 mm-1,5 mm, de préférence dans une plage de 0,4 mm-1,2 mm, un diamètre intérieur (DI) de l'ordre de 0,15 mm-1,4 mm et/ou une longueur (l) de l'ordre de 90 mm-500 mm de préférence de l'ordre de 90-300 mm et d'une manière particulièrement préférentielle, de l'ordre de 90 mm-120 mm.

10. Canule selon l'une des revendications précédentes,
**caractérisée en ce que**
le revêtement (18) a une épaisseur (d) de l'ordre de 0,1 µm-30 µm et d'une manière particulièrement préférentielle, de l'ordre de 0,1 µm-15 µm.

11. Procédé de fabrication d'une canule (1, 1') notamment pour une ponction et injection lombaires selon l'une des revendications précédentes,
**caractérisé par** les étapes de procédé consistant à :
- fournir le tube de canule (10) ayant un côté intérieur (11), un côté extérieur (12), une première zone d'extrémité ouverte et une seconde zone d'extrémité,
- réaliser une ouverture (20) notamment par un procédé avec enlèvement de copeaux, et
- revêtir le côté intérieur (11) du tube de canule (10) au moins autour de l'ouverture (20),
procédé **caractérisé en ce que**
on revêt tout le côté intérieur (11) et le côté extérieur (12) du tube de canule (10) ainsi que l'arête intérieure de l'ouverture (20).

12. Procédé de fabrication selon la revendication 11,
**caractérisé en ce que**
la seconde zone d'extrémité a une pointe (14) fermée et sensiblement conique et l'ouverture (20) est réalisée au voisinage de la pointe (14).

13. Procédé de fabrication selon la revendication 11,
**caractérisé en ce que**
le tube de canule (10) comporte dans sa seconde zone d'extrémité, une coupe en biais qui forme une pointe (14) et l'ouverture (20) est prévue côté frontal dans la seconde zone d'extrémité du tube de canule (10).

14. Procédé de fabrication selon l'une des revendications 11 à 13,
**caractérisé en ce que**
avant de revêtir, on forme notamment par injection une embase (30) à la seconde zone d'extrémité sur le tube de canule (10).
